# EUROPEAN PATENT SPECIFICATION

(11) **EP 3 733 192 B1**
(45) Date of publication and mention of the grant of the patent: **24.07.2024**
(21) Application number: 20180024.0
(22) Date of filing: 29.07.2009
(51) Int. Cl.: A61K 31/722, A61K 47/36, A61K 31/506, A61K 31/56, A61K 31/585, A61P 17/14, A61K 8/34, A61K 8/49, A61K 8/63, A61K 8/73, A61K 8/9741, A61K 8/9789, A61K 8/9794, A61K 9/00, A61P 17/00, A61P 17/18, A61P 43/00, A61Q 5/00, A61Q 7/00

(54) **FINASTERIDE FORMULATIONS FOR DRUG RELEASE TO HAIR AND SCALP**
FINASTERID-FORMULIERUNGEN ZUR WIRKSTOFFFREISETZUNG AN HAAR UND KOPFHAUT
FORMULATIONS DE FINASTÉRIDE POUR LA LIBÉRATION DE MÉDICAMENTS DANS LES CHEVEUX ET LE CUIR CHEVELU

(30) Priority: 04.08.2008 EP 08161757
(43) Date of publication of application: 04.11.2020
(62) Divisional of application: 09804542.0
(73) Proprietor: Polichem SA, L-1526 Luxembourg (LU)
(72) Inventor: MAILLAND, Federico, CH-6900 Lugano (CH); MURA, Emanuela, I-22100 Como (IT)
(74) Representative: J A Kemp LLP

(56) References cited:
- EP-A1- 1 491 202
- WO-A2-2007/099398
- US-A1- 2002 192 280
- BIRUSS ET AL: "Skin permeation of different steroid hormones from polymeric coated liposomal formulations", EUROPEAN JOURNAL OF PHARMACEUTICS AND BIOPHARMACEUTICS, ELSEVIER SCIENCE PUBLISHERS B.V., AMSTERDAM, NL, vol. 62, no. 2, 1 February 2006 (2006-02-01), pages 210 - 219, XP005269406, ISSN: 0939-6411

## Description

The present invention relates to liquid compositions containing hydroxypropyl chitosan, a chitosan derivative, for the preparation of a medicament, or a medical device, or a sanitary product, or a cosmetic, forming a film after application onto the scalp, or onto the hair, useful for delivery of actives onto the scalp surface and on the hair. Delivery of active ingredients to scalp or the hair, contained in drugs, sanitary products, detergents or cosmetics, often constitutes a problem in that the common formulations, like cream, ointment, gel, powder, or foam, do not allow a long lasting contact with the scalp or hair surface.

Chitosan and its derivatives are amino-polysaccharides, derived from the chitin extracted from the exoskeleton of the crustaceans, known in the art for their use in different preparations. KR20020084672 discloses chitosan as an ingredient of microspheres, useful as a carrier for separation of proteins or peptides; KR20020048534 reports chitosan as an ingredient of a pack composition for skin massage, including paraffin wax as an effective component; JP2005306746 is teaching the use of chitosan to obtain a wrinkle therapeutic agent as an ingredient of gel-like or spongy preparations of botulinus toxin. WO2005055924 reports chitosan derivatives as ingredients of hydrogels useful for cavity-filling wound dressings. JP2004231604 teaches compositions of chitosans having a high deacetylation degree, as an ingredient of a carrier sheet with a porous spongy texture. WO03042251 discloses compositions comprising chitosan in the form of a network of nano-sized fibres. WO02057983 discloses a multilayered, air gap sheet of chitosan with a regular lamellar structure which retains drugs for a prolonged period of time; JP11060605 teaches an amphiphilic chitosan derivative which can be used as dispersion stabilizer or emulsifier in a drug for application to skin. Finally, EP1303249, discloses a nail varnish composition containing at least one antimycotic agent and an hydroxyalkyl or a carboxyalkyl chitosan, whereas WO2004/112814 discloses a nail restructuring composition based on one herb extract from the genus Equisetum in combination with hydroxypropyl chitosan.

Biruss et al., European Journal of Pharmaceutics and Biopharmaceutics, 62, 2006, pages 210-219, discusses skin permeation of different steroid hormones from polymeric coated liposomal formulations. Various polymers, including chitosan-EDTA, are studied.

US 2002/192280 discloses compositions, which may comprise a chitosan or chitosan derivative, for use in treating a variety of inflammatory conditions. WO 2007/099398 discloses compositions for use in the treatment of human skin, including facial skin and the scalp, to alleviate the symptoms of cosmetic or dermatologic skin conditions.

It has now surprisingly been found that liquid preparations of chitosan derivatives, besides being useful for the application to nails, may form an elastic film, after application to the scalp and after evaporation of the volatile solvents, suitable to maintain drugs and other actives in intimate contact to the scalp and/or hair surface. The film forming solutions containing chitosans, pharmaceutically active agents and volatile solvents, may easily be sprayed onto the scalp or hair surface, by allowing quick evaporation of the volatile solvents and easy formation of an elastic film, that avoids, as an example, bothersome sensation of oily scalp compared to creams, lotions and ointments, and avoids long time waiting for drying, when large scalp surface is to be treated. The film forming solutions of chitosan or chitosan derivatives may also be applied on the scalp by gently massage, or by spray. The film forming solutions of chitosans allow quick penetration of actives into the deep layers of the scalp, thus resulting useful for the safe treatment and/or prevention of scalp conditions and/or diseases, like hair loss, where penetration of the active ingredient until the hair follicles is needed. Moreover, the dermal film formed after evaporation of solvents of liquid chitosan compositions allows long lasting intimate contact with the scalp and/or hair surface, and continuous release of drugs or other actives for many hours after the application.

### DESCRIPTION OF THE INVENTION

The object of the present invention is a composition comprising: 0.25 wt% finasteride; 55.00 wt% ethyl alcohol 96°; 5.00 wt% propylene glycol; 1.00 wt% hydroxypropyl chitosan; and 38.75 wt% purified water, for use in the treatment and/or prevention of scalp and/or hair conditions and/or diseases.

The film forming solutions of hydroxypropyl chitosan are topically applied to the scalp and/or hair of the recipient, preferably a human being; they may be applied by a gently massage on the scalp or may easily be sprayed by allowing the formation of an elastic film. The film forming solutions of hydroxypropyl chitosan allow quick penetration of actives, a long lasting intimate contact with the scalp and continuous release of drugs for many hours after the application.

Liquid preparations of this invention may be in the form of solutions. Percentages by weight are given with respect to the whole preparation. The active pharmaceutical agent, which is finasteride, is suitable to form an elastic film after evaporation of solvent, said film being in intimate contact with the scalp surface and/or of the hair and allowing quick and long lasting penetration of said actives useful for treating scalp and/or hair conditions and/or diseases, like hair loss, baldness, alopecia, androgenetic alopecia, hair fragility and other hair conditions. Compositions object of this invention are superior to the conventional formulations, in that they can be sprayed onto the surface, leaving an uniform and invisible film. Moreover, compositions according to the present invention do not dirty, do not dry like gels and lotions do, and do not give bothersome sensation when applied, like other rigid film preparations do. Pharmaceutical compositions are prepared according to conventional technique, using compatible excipients and pharmaceutically acceptable carriers, and may contain, in combination, other active principles with complementary or, in any case, useful activity.

Examples of these compositions prepared according to the present invention include: solutions, emulsions, suspensions, colloids, for application to nude or hairy scalp.

The active agent, finasteride, is a 5-alpha reductase inhibitor and is suitable to prevent and to treat hair loss, baldness, alopecia; to nourish, volumize and reinforce the hair.

The compositions according to the invention may be applied onto the scalp surface by gently massage of the concerned area, or onto the hair by spray. After evaporation, an elastic film is formed onto the treated surface, that allows the continuous delivery of the actives to the scalp and/or the hair for many hours or even for days.

The pharmaceutical compositions and the uses of the present invention will now be more fully described by the following examples. It should, however, be noted that such examples are given by way of illustration and not of limitation.

### EXAMPLE 1

A film forming solution having the following composition wt./wt.% was prepared:

| | |
|---|---|
| 1) Finasteride | 0.25% |
| 2) Ethyl Alcohol 96° | 55.00% |
| 3) Propylene Glycol | 5.00% |
| 4) Hydroxypropyl Chitosan | 1.00% |
| 5) Purified water | 38.75% |

### Preparation

Ethyl Alcohol, Propylene Glycol and water were mixed at room temperature. Finasteride was then added and mixed until a clear solution was obtained. Hydroxypropyl Chitosan was added as the final ingredient, and the mixture was stirred at room temperature for 24 hours or until dissolution.

The obtained formulation was a clear and colourless solution, homogenous in appearance even after prolonged storage.

Moreover, the liquid was able to form a matte, non-sticky and elastic film, which could strongly adhere to the scalp surface.

### EXAMPLE 2

An in vitro permeation test was performed by applying the film forming solution according to the Example 1 to excised hairless rat skin, obtained from dorsal or abdominal skin of male hairless rats. Portions of the skin (ca 9 cm²), after removal of adhering fat and subcutaneous tissues, were placed as a barrier between the two compartment of Summer permeation vertical cells (Gummer, CL. et al. The skin penetration cell: design update. Int. J. Pharm. 1987, 40, 101-104). The receiving phase was introduced into the lower compartment and 1.0 or 0.5 mL of the composition according to the Example 1 were regularly distributed on the exposed skin surface. At predetermined time intervals (2, 4, 8, 12, 16, 20 and 24 hours) 5.0 mL of the receiving solution were collected for analysis and immediately replaced by an equal volume of fresh buffer. The experiment was replicated 3 times.

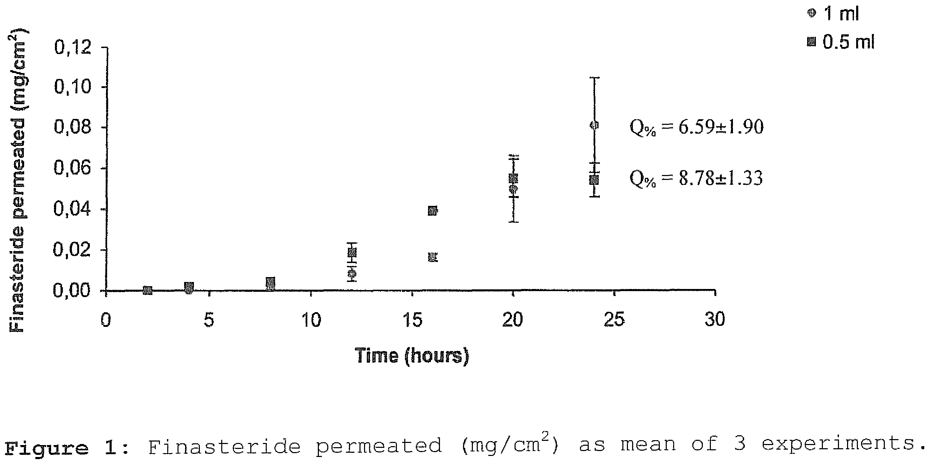

The finasteride permeated through hairless rat skin in the 3 experiments is reported in Figure 1. The total percent quantity (Q%) permeated through the rat hairless skin was 6.59 ± 1.90% for the 1.0 mL dose and 8.78 ± 1.33% for the 0.5 mL dose.

It is concluded that finasteride was able to permeate the rat skin, after the application of the film forming solution of hydroxypropyl chitosan according to the Example 1, in a quick and long lasting way.

## Claims

1. A composition comprising:
- 0.25 wt% finasteride;
- 55.00 wt% ethyl alcohol 96°;
- 5.00 wt% propylene glycol;
- 1.00 wt% hydroxypropyl chitosan; and
- 38.75 wt% purified water,
for use in the treatment and/or prevention of scalp and/or hair conditions and/or diseases.

2. A composition for use according to claim 1, wherein the composition is a liquid.

3. A composition for use according to claim 1 or claim 2, wherein the composition is applied as a spray.

4. A composition for use according to any one of claims 1 to 3, wherein said scalp and/or hair conditions and/or diseases are selected from hair loss, baldness, alopecia, androgenetic alopecia and hair fragility.

## Patentansprüche

1. Zusammensetzung, die umfasst:
0,25 Gew.-% Finasterid;
55,00 Gew.-% Ethylalkohol 96°;
5,00 Gew.-% Propylenglykol;
1,00 Gew.-% Hydroxypropylchitosan; und
38,75 Gew.-% gereinigtes Wasser,
zur Verwendung bei der Behandlung und/oder Vorbeugung von Kopfhaut- und/oder Haarzuständen und/oder -krankheiten.

2. Zusammensetzung zur Verwendung nach Anspruch 1, wobei die Zusammensetzung eine Flüssigkeit ist.

3. Zusammensetzung zur Verwendung nach Anspruch 1 oder Anspruch 2, wobei die Zusammensetzung als Spray angewendet wird.

4. Zusammensetzung zur Verwendung nach einem der Ansprüche 1 bis 3, wobei die Kopfhaut- und/oder Haarzustände und/oder Krankheiten ausgewählt sind aus Haarausfall, Kahlheit, Alopezie, androgenetischer Alopezie und Haarbrüchigkeit.

## Revendications

1. Composition, comprenant :
- 0,25 % en poids de finastéride ;
- 55,00 % en poids d'alcool éthylique à 96 ° ;
- 5,00 % en poids de propylène glycol ;
- 1,00 % en poids d'hydroxypropyl-chitosane ; et
- 38,75 % en poids d'eau purifiée,
pour utilisation dans le traitement et/ou la prévention de conditions et/ou de maladies du cuir chevelu et/ou des cheveux.

2. Composition pour utilisation selon la revendication 1, dans laquelle la composition est un liquide.

3. Composition pour utilisation selon la revendication 1 ou la revendication 2, dans laquelle la composition est appliquée sous forme de pulvérisation.

4. Composition pour utilisation selon l'une quelconque des revendications 1 à 3, dans laquelle lesdites conditions et/ou maladies du cuir chevelu et/ou des cheveux sont sélectionnées parmi : perte des cheveux, calvitie, alopécie, alopécie androgénétique et fragilité des cheveux.
